# EUROPEAN PATENT APPLICATION

(11) **EP 4 667 586 A2**
(43) Date of publication of application: **24.12.2025**
(21) Application number: 25212760.0
(22) Date of filing: 18.06.2020
(51) Int. Cl.: C12Q 1/6869

(54) **COMPOSITIONS AND METHODS FOR PREPARING NUCLEIC ACID SEQUENCING LIBRARIES USING CRISPR/CAS9 IMMOBILIZED ON A SOLID SUPPORT**

(30) Priority: 12.07.2019 US 201962873609 P
(62) Divisional of application: 20733788.2
(71) Applicant: Illumina Cambridge Limited, Cambridge CB21 6DF (GB)
(72) Inventor: GORMLEY, Niall, Anthony, Cambridge CB21 6DF (GB)
(74) Representative: Murgitroyd & Company

(57) **Abstract**

Presented are methods and compositions for using immobilized CRISPR/Cas9 enzymes for generating an immobilized library of randomly fragmented, double-stranded target nucleic acid fragments on a surface. The methods are useful for generating nucleic acid fragments for use in a variety of processes, including massively parallel nucleic acid sequencing.

## Description

### CROSS-REFERENCE TO RELATED APPLICATION

This application claims the benefit of priority of US Provisional Application No. 62/873,609, filed July 12, 2019, which is incorporated by reference herein in its entirety for any purpose.

### DESCRIPTION

### FIELD

This application relates to methods for using CRISPR/Cas9 enzymes immobilized on a solid support to generate a library of randomly fragmented, double-stranded nucleic acid fragments, solid supports comprising the immobilized CRISPR/Cas9 enzyme, and related compositions. The immobilized library of randomly fragmented, double-stranded nucleic acid fragments are useful as templates, *e.g.,* for a variety of applications including, *e.g.,* high throughput, massively parallel and/or multiplex nucleic acid sequencing.

### BACKGROUND

There are a variety of methods and applications for which it is desirable to generate a library of randomly fragmented nucleic acid molecules from target double-stranded nucleic acid target molecules, such as double-stranded DNA (dsDNA) target molecules. Often, the purpose is to generate smaller nucleic acid molecules (*e.g.,* nucleic acid fragments) from larger double-stranded nucleic molecules for use as templates in nucleic acid sequencing reactions.

Many of the methods currently used for fragmentation and tagging of double-stranded nucleic acids for use in next-generation sequencing are wasteful of the nucleic acid, require expensive instruments for fragmentation, and the procedures for fragmentation, tagging and recovering tagged nucleic fragments are difficult, tedious, laborious, time-consuming, inefficient, costly, require relatively large amounts of sample nucleic acids. In addition, many of these methods generate nucleic acid fragments that are not fully representative of the sequences contained in the sample nucleic acids from which they were generated. Thus, what is needed in the art are methods that provide speed and ease of use when generating libraries of randomly fragmented, double stranded nucleic acid fragments from target double-stranded nucleic acid and which can be easily applied to nucleic acid analysis methods such as next-generation sequencing and amplification methods.

### SUMMARY

In accordance with the description, this application describes a method of preparing an immobilized library of randomly fragmented, double-stranded nucleic acid fragments comprising: (a) providing a solid support having CRISPR/Cas9 enzymes immobilized thereon; and (b) applying a target double-stranded nucleic acid to the solid support under conditions whereby the target double-stranded nucleic acid is randomly fragmented by the CRISPR/Cas9 enzymes, and the CRISPR/Cas9 binds at least one strand of the double-stranded nucleic acid fragments; thereby producing an immobilized library of randomly fragmented, double-stranded nucleic acid fragments.

In some embodiments, the target double-stranded nucleic acid comprises double-stranded DNA (dsDNA), double-stranded RNA (dsRNA), or a double-stranded RNA/DNA hybrid. In some embodiments, the target double-stranded nucleic acid is dsDNA.

In some embodiments, the CRISPR/Cas9 enzymes are bound to a first polynucleotide that directs the CRISPR/Cas9 enzymes to bind the target double-stranded nucleic acid in a non-sequence specific manner. In some embodiments, the first polynucleotide is immobilized to the solid support. In some embodiments, the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner.

In some embodiments, the sgRNA comprises (GC)ₙ or (AT)ₙ, wherein n is 5-20, 10-15, or 10.

In some embodiments, the sgRNA is 10 to 40, 15 to 35, or 17 to 20 nucleotides.

In some embodiments, the first polynucleotide is biotinylated and the solid support comprises one or more biotin binding proteins. In some embodiments, the biotin binding proteins comprise avidin, streptavidin, neutravidin, an anti-biotin antibody, a biotin receptor, and/or a biotin-binding enzyme. In some embodiments, the biotin-binding enzyme comprises biotinidase or biotin holocarboxylase synthetase.

In some embodiments, the method comprises washing the solid support with the double-stranded nucleic acid fragments immobilized thereon to remove any unbound nucleic acids.

In some embodiments, the method comprises amplifying the double-stranded nucleic acid fragments immobilized on the solid surface. In some embodiments, the amplifying comprises providing a polymerase and an amplification primer corresponding to a portion of the first polynucleotide.

In some embodiments, applying the target double-stranded nucleic acid to the solid support comprises treating the CRISPR/Cas9 enzymes with one or more reagents that reduce the nucleic acid binding specificity of the CRISPR/Cas9 enzymes.

In some embodiments, the one or more reagents comprise betaine, dimethyl sulfoxide (DMSO), ethanol, ethylene glycol, dimethylacetamide, dimethylformamide, and/or sulphalane.

In some embodiments, the CRISPR/Cas9 enzymes are present on the solid support at a density of at least 10³, 10⁴, 10⁵, or 10⁶ enzymes per mm².

In some embodiments, the lengths of the double-stranded nucleic acid fragments in the immobilized library are proportional to the density of CRISPR/Cas9 enzymes on the solid support.

In some embodiments, the solid support comprises microparticles, a patterned surface, or wells. In some embodiments, the microparticles are beads.

In some embodiments, the method comprises: (c) applying an intercalating dye to at least a portion of the immobilized library of double-stranded nucleic acid fragments to obtain a set of stained immobilized fragments; and obtaining an image of the stained immobilized fragments.

In some embodiments, applying a target double-stranded nucleic acid comprises adding a biological sample to the solid support. In some embodiments, the biological sample comprises a cell lysate. In some embodiments, the biological sample comprises whole cells. In some embodiments, the biological sample is selected from the group consisting of blood, plasma, serum, lymph, mucus, sputum, urine, semen, cerebrospinal fluid, bronchial aspirate, feces, and macerated tissue.

In some embodiments, the method comprises tagging the double-stranded nucleic acid fragments.

In some embodiments, the double-stranded nucleic acid fragments are tagged with a first tag comprising a first tag domain. In some embodiments, first tag domain comprises a region for cluster amplification. In some embodiments, the first tag domain comprises a region for priming a sequencing reaction.

In some embodiments, the method comprises liberating the immobilized double-stranded nucleic acid fragments from the solid support. In some embodiments, the liberating comprises cleavage of the CRISPR/Cas9 enzymes from the solid support. In some embodiments, the liberating comprises performing polymerase chain reaction (PCR), strand displacement amplification (SDA), transcription mediated amplification (TMA) and nucleic acid sequence based amplification (NASBA), or other amplification process. In some embodiments, the PCR comprises suppression PCR. In some embodiments, the liberating comprises applying light or heat.

This application also describes a method of preparing an immobilized library of randomly fragmented, double-stranded nucleic acid fragments comprising: (a) providing a solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner, and wherein the biotinylated first polynucleotide is bound to a biotin binding protein on the solid support; and (b) applying a target double-stranded nucleic acid to the solid support under conditions whereby the target double-stranded nucleic acid is randomly fragmented by the CRISPR/Cas9 complexes, and the CRISPR/Cas9 complexes bind at least one strand of the double-stranded nucleic acid fragments; thereby producing an immobilized library of randomly fragmented, double-stranded nucleic acid fragments.

In some embodiments, the biotin binding protein comprises avidin, streptavidin, neutravidin, an anti-biotin antibody, a biotin receptor, and/or a biotin-binding enzyme. In some embodiments, the biotin-binding enzyme comprises biotinidase or biotin holocarboxylase synthetase.

In some embodiments, applying the target double-stranded nucleic acid to the solid support comprises treating the CRISPR/Cas9 enzymes with one or more reagents that reduce the nucleic acid binding specificity of the CRISPR/Cas9 enzymes. In some embodiments, the one or more reagents comprise betaine, dimethyl sulfoxide (DMSO), ethanol, ethylene glycol, dimethylacetamide, dimethylformamide, and/or sulphalane.

In some embodiments, the method comprises tagging the double-stranded nucleic acid fragments. In some embodiments, the double-stranded nucleic acid fragments are tagged with a first tag comprising a first tag domain. In some embodiments, first tag domain comprises a region for cluster amplification. In some embodiments, the first tag domain comprises a region for priming a sequencing reaction.

In some embodiments, the method comprises liberating the immobilized double-stranded nucleic acid fragments from the solid support. In some embodiments, the liberating comprises cleavage of the CRISPR/Cas9 enzymes from the solid support. In some embodiments, the liberating comprises performing PCR or other amplification process. In some embodiments, the PCR comprises suppression PCR. In some embodiments, the liberating comprises applying light or heat.

This application also describes a solid support having a library of double-stranded nucleic acid fragments immobilized thereon prepared according to the methods described herein.

This application also describes a solid support having CRISPR/Cas9 complexes immobilized thereon, wherein the CRISPR/Cas9 complexes comprise CRISPR/Cas9 enzymes that randomly fragment a target double-stranded nucleic acid.

In some embodiments, the target double-stranded nucleic acid comprises double-stranded DNA (dsDNA), double-stranded RNA (dsRNA), or a double-stranded RNA/DNA hybrid. In some embodiments, the target double-stranded nucleic acid is dsDNA.

In some embodiments, CRISPR/Cas9 enzymes are bound to a first polynucleotide that directs the CRISPR/Cas9 enzymes to bind the target double-stranded nucleic acid in a non-sequence specific manner.

In some embodiments, the first polynucleotide is immobilized to the solid support.

In some embodiments, the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner.

In some embodiments, the sgRNA comprises (GC)ₙ or (AT)ₙ, wherein n is 5-20 or 10-15. In some embodiments, n is 10.

In some embodiments, the sgRNA is 10 to 40 or 15 to 30 nucleotides. In some embodiments, the sgRNA is 17 or 20 nucleotides.

In some embodiments, the first polynucleotide is biotinylated and the solid support comprises biotin binding proteins. In some embodiments, the biotin binding proteins comprise avidin, streptavidin, neutravidin, an anti-biotin antibody, a biotin receptor, and/or a biotin-binding enzyme. In some embodiments, the biotin-binding enzyme comprises biotinidase or biotin holocarboxylase synthetase

In some embodiments, the CRISPR/Cas9 enzymes are present on the solid support at a density of at least 10³, 10⁴, 10⁵, or 10⁶ enzymes per mm².

In some embodiments, the solid support comprises microparticles, a patterned surface, or wells. In some embodiments, the microparticles are beads.

The application also describes a composition comprising a solid support described herein and one or more reagents that reduce the nucleic acid binding specificity of the CRISPR/Cas9 enzymes. In some embodiments, the one or more reagents comprise betaine, dimethyl sulfoxide (DMSO), ethanol, ethylene glycol, dimethylacetamide, dimethylformamide, and/or sulphalane.

The application also describes a solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind a target double-stranded nucleic acid in a non-sequence specific manner, and wherein the biotinylated first polynucleotide is bound to biotin binding protein on the solid support.

In some embodiments, the biotin binding protein comprises avidin, streptavidin, neutravidin, an anti-biotin antibody, a biotin receptor, and/or a biotin-binding enzyme. In some embodiments, the biotin-binding enzyme comprises biotinidase or biotin holocarboxylase synthetase.

In some embodiments, the CRISPR/Cas9 complexes are present on the solid support at a density of at least 10³, 10⁴, 10⁵, 10⁶ complexes per mm².

In some embodiments, the solid support comprises microparticles, a patterned surface, or wells. In some embodiments, the microparticles are beads.

The application also describes a composition comprising a solid support described herein and one or more reagents that reduce the nucleic acid binding specificity of the CRISPR/Cas9 enzymes. In some embodiments, the one or more reagents comprise betaine, dimethyl sulfoxide (DMSO), ethanol, ethylene glycol, dimethylacetamide, dimethylformamide, and/or sulphalane.

Additional objects and advantages will be set forth in part in the description which follows, and in part will be obvious from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

The accompanying drawing, which is incorporated in and constitutes a part of this specification, illustrates one embodiment and together with the description, serves to explain the principles described herein.

### BRIEF DESCRIPTION OF THE DRAWING

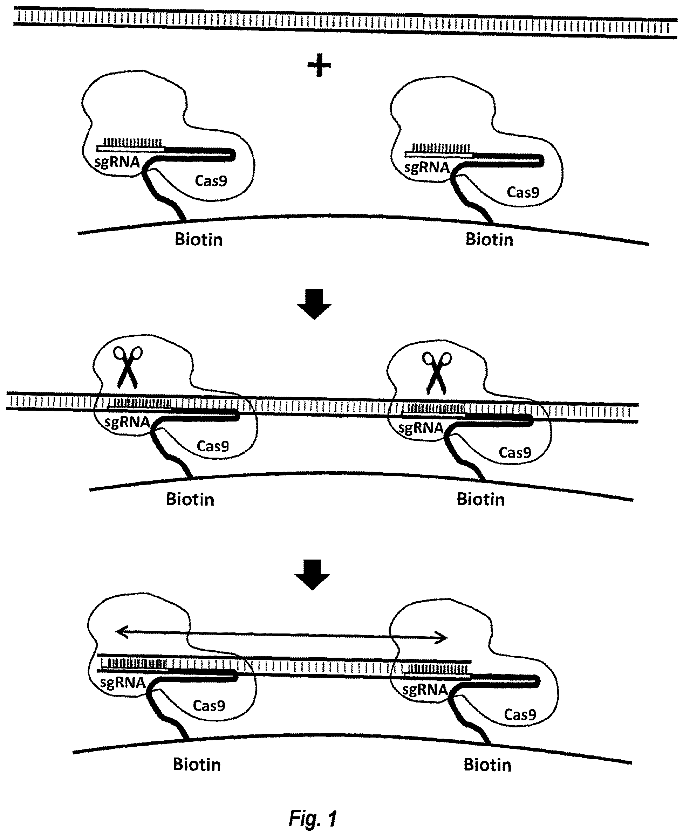

Figure 1 shows one embodiment of the invention. In this embodiment, CRISPR/Cas9 complexes are immobilized on a surface. The CRISPR/Cas9 complexes have a CRISPR/Cas9 (Cas9) enzyme and a biotinylated polynucleotide comprising a 3' portion comprising a CRISPR/Cas9 end sequence and a 5' portion comprising a single-stranded guide RNA (sgRNA). A target double-stranded nucleic acid is applied to the solid support, randomly fragmented by the CRISPR/Cas9 complexes, and the double-stranded nucleic acid fragments are bound to the CRISPR/Cas9 complexes immobilized on the surface. The size of the nucleic acid fragment depends upon the distance between the immobilized CRISPR/Cas9 enzymes on the surface.

### DESCRIPTION OF THE EMBODIMENTS

Current protocols for sequencing nucleic acid samples routinely employ a sample preparation process that converts nucleic acid, such as DNA, or RNA, or a RNA/DNA hybrid into a library of templates. These methods can result in loss of nucleic acid sample and often require expensive instruments for fragmentation. In addition, the sample preparation methods are often difficult, tedious, and inefficient.

In standard sample preparation methods, each template contains an adaptor at either end of the insert and often a number of steps are required to both modify the nucleic acid and to purify the desired products of the modification reactions. These steps are performed in solution prior to the addition of the adapted fragments to a flow cell where they are coupled to the surface by a primer extension reaction that copies the hybridized fragment onto the end of a primer covalently attached to the surface. These 'seeding' templates then give rise to monoclonal clusters of copied templates through several cycles of amplification.

The number of steps required to transform a target double-stranded nucleic acid, such as DNA, into randomly fragmented, double-stranded nucleic acid fragments in solution ready for cluster formation and sequencing can be minimized by the use of CRISPR/Cas9 enzyme mediated fragmentation. Following a purification step to remove any unbound nucleic acids, additional sequences are added to the ends of the nucleic acid fragments by PCR.

Solution-based fragmentation has drawbacks and requires several labor-intensive steps. Additionally, bias can be introduced during polymerase chain reaction (PCR) amplification steps. The methods and compositions presented herein overcome those drawbacks and allow unbiased sample preparation, cluster formation and sequencing to occur on a single solid support with minimal requirements for sample manipulation or transfer.

The present disclosure relates to the surprising discovery that CRISPR/Cas9 enzymes pre-coupled to the surface of a solid support, such as a flow cell, can be used under conditions to effectively randomly fragment and immobilize intact target nucleic acids on the solid support. In specific embodiments, one or more of the strands that comprise the CRISPR/Cas9 enzymes are attached to the surface of the solid support via their 5' end. The CRISPR/Cas9 enzyme can also be encompassed within a complex that contains sequences that enable subsequent cluster generation and sequencing.

The methods and compositions presented herein provide several advantages over solution-based fragmentation methods and less random fragmentation methods. For example, purified, partially purified or even unpurified intact target nucleic acid can be loaded directly onto a solid support or flow cell for generation of clusters, without prior sample preparation. In addition, the contiguity of sequence information in the original intact nucleic acid can be physically preserved by the juxtaposition of fragments on the surface of the solid support or flow cell. As a further advantage, nucleic acid fragments are physically linked to the surface of the solid support or flow cell so purification of reagents following further manipulation of the nucleic acid fragments can be achieved by flow-through buffer exchange, e.g., in the flow cell channel.

Further, improved random fragmentation of the target double-stranded nucleic acid allows the library of randomly fragmented, double-stranded nucleic acid fragments to be better representative of the original sample.

### I. Random Fragmentation on a Solid Support

In accordance with the above, presented herein are methods of preparing an immobilized library of randomly fragmented, double-stranded nucleic fragments, and solid supports having a library of double-stranded nucleic acid fragments immobilized thereon prepared according to such methods. In some embodiments, the methods comprise: (a) providing a solid support having CRISPR/Cas9 enzymes immobilized thereon; and (b) applying a target double-stranded nucleic acid to the solid support under conditions whereby the target double-stranded nucleic acid is randomly fragmented by the CRISPR/Cas9 enzymes, and the CRISPR/Cas9 binds at least one strand of the double-stranded nucleic acid fragments; thereby producing an immobilized library of randomly fragmented, double-stranded nucleic acid fragments.

In some embodiments, the CRISPR/Cas9 enzymes are directly immobilized onto the solid support.

In some embodiments, the methods comprise: (a) providing a solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind the target double-stranded nucleic acid in a non-sequence specific manner, and wherein the biotinylated first polynucleotide is bound to a biotin binding protein on the solid support; and (b) applying a target double-stranded nucleic acid to the solid support under conditions whereby the target double-stranded nucleic acid is randomly fragmented by the CRISPR/Cas9 complexes, and the CRISPR/Cas9 complexes bind at least one strand of the double-stranded nucleic acid fragments; thereby producing an immobilized library of randomly fragmented, double-stranded nucleic acid fragments.

As used herein, the term "randomly fragmented" refers to fragmentation of a target nucleic acid in a random manner to produce a range of fragments (e.g., fragments generated by fragmentation at random locations from a sequence perspective). In some embodiments, the target nucleic acid is randomly fragmented in a non-sequence specific manner to produce a range of fragment identities. Fragmentation is controllable with the present invention, both in terms of normalized product quantities and fragment size (i.e., controllable size selection), irrespective of input quantity of DNA. For example, as shown in Figure 1, the distance between neighboring CRISPR/Cas9 enzymes on the solid supports can dictate the size range of fragments that become immobilized on the surface of the solid support.

In some embodiments, the target nucleic acid is fragmented in a sequence specific manner. For example, as discussed below, two CRISPR/Cas9 enzyme complexes on the same solid support can include a pair of sgRNA that flank a region of interest in the target nucleic acid. In such embodiments, a single fragment is produced by each pair of sgRNA. In other embodiments, a population of solid supports (e.g., beads) is used and each solid support in the population of solid supports can carry a different pair of sgRNA. In this manner, different solid supports would target different sequences in the target nucleic acid. In some embodiments, the different sgRNA pairs target different target sequences in the genome. These methods enable multiplex targeting.

As used herein, the term "CRISPR/Cas9 enzymes" refer generally to Clustered Regularly Interspaced Short Palindromic Repeats (CRISPR) and CRISPR-associated protein-9 nuclease (Cas9) enzymes. Cas9, also known as Csn1, is a CRISPR-associated protein containing two nuclease domains, a RuvC nuclease domain and an HNH nuclease domain, that is programmed by small RNAs to cleave nucleic acid (e.g., DNA). CRISPR/Cas9 enzymes are generally known to those of skill in the art, as exemplified by the disclosure of US Pat. App. Publ. No. 2019/0202856, the content of which is incorporated herein by reference in its entirety. For example, the engineered CRISPR/Cas9 enzymes can be derived from Streptococcus pyogenes Cas9 (SpCas9), Streptococcus thermophilus Cas9 (StCas9), Streptococcus pasteurianus (SpaCas9), Campylobacter jejuni Cas9 (CjCas9), Francisella novicida Cas9 (FnCas9), or Neisseria cinerea Cas9 (NcCas9). Additional variants of Cas9 known to cleave nucleic acids are known to those skilled in the art, and include wild-type or naturally occurring Cas9 and mutant or modified Cas9 (e.g., Cas9D10A). It will be appreciated that any CRISPR/Cas9 enzyme that is capable of fragmenting or cleaving a target nucleic acid can be used in the present invention under conditions by which the CRISPR/Cas9 enzyme is capable of randomly fragmenting or cleaving the target DNA.

As used herein, the term "CRISPR/Cas9 enzyme complex" refers generally to a CRISPR/Cas9 enzyme bound to a first polynucleotide that directs the CRISPR/Cas9 enzyme to bind the target double-stranded nucleic acid in a non-sequence specific or sequence-specific manner. In some embodiments, the first polynucleotide is immobilized to the solid support. In some embodiments, the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific or sequence specific manner. In some embodiments, the first polynucleotide is biotinylated and the solid support comprises one or more biotin binding proteins. For example, the CRISPR/Cas9 enzyme complex may comprise a CRISPR/Cas9 enzyme bound to biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific or sequence specific manner, and wherein the biotinylated first polynucleotide is bound to a biotin binding protein on the solid support.

As used herein, the term "double-stranded nucleic acid" refers to a double-stranded DNA (dsDNA), double-stranded RNA (dsRNA), or a double-stranded RNA/DNA hybrid. In some embodiments, the target double-stranded nucleic acid is dsDNA. Although the term "DNA" is used throughout the present disclosure in connection with the target double-stranded nucleic acid molecule, it should be understood that any suitable nucleic acid or nucleic acid analogue can be randomly fragmented.

The term "CRISPR/Cas9 end" refers to a double-stranded nucleic acid that exhibits only the nucleotide sequences (the "CRISPR/Cas9 end sequences") that are necessary to form the complex with the CRISPR/Cas9 enzyme. CRISPR/Cas9 ends can comprise any nucleic acid or nucleic acid analogue suitable for forming a functional complex with the CRISPR/Cas9 enzyme. For example, the CRISPR/Cas9 end can comprise DNA, RNA, modified bases, non-natural bases, modified backbone, and can comprise nicks in one or both strands.

The terms "tag" and "tag domain" as used herein refer to a portion or domain of a polynucleotide that exhibits a sequence for a desired intended purpose or application. Tag domains can comprise any sequence provided for any desired purpose. In some embodiments, a tag comprises one or more functional sequences selected from the group consisting of universal sequences, primer sequences, index sequences, capture sequences, barcode sequences (used, e.g., for counting or error correction), cleavage sequences, sequencing-related sequences, sequences for enrichment, and combinations thereof. For example, in some embodiments, a tag domain comprises one or more restriction endonuclease recognition sites. In some embodiments, a tag domain comprises one or more regions suitable for hybridization with a primer for a cluster amplification reaction. In some embodiments, a tag domain comprises one or more regions suitable for hybridization with a primer for a sequencing reaction. It will be appreciated that any other suitable feature can be incorporated into a tag domain. In some embodiments, the tag domain comprises a sequence having a length between 5 and 200 bp. In some embodiments, the tag domain comprises a sequence having a length between 10 and 100 bp. In some embodiments, the tag domain comprises a sequence having a length between 20 and 50 bp. In some embodiments, the tag domain comprises a sequence having a length between 5, 6, 7, 8, 9, 10, 20, 30, 40, 50, 60, 70, 80, 90, 100, 150 and 200 bp.

In the methods and compositions presented herein, CRISPR/Cas9 enzymes are immobilized to the solid support. In some embodiments, the CRISPR/Cas9 enzymes are immobilized to the support via one or more polynucleotides. In some embodiments, the one or more polynucleotides, such as a first polynucleotide, directs the CRISPR/Cas9 enzymes to bind the target double-stranded nucleic acid in a non-sequence specific manner (i.e., a condition which directs the CRISPR/Cas9 enzyme to randomly fragment or cleave the target double-stranded nucleic acid). In some embodiments, the one or more polynucleotides direct the CRISPR/Cas9 enzymes to bind the target double-stranded nucleic acid in a sequence-specific manner and fragmentation is achieved by using a population of solid supports where each solid support comprises a first CRISPR/Cas9 complex comprising a first polynucleotide that directs the CRISPR/Cas9 enzyme to bind 3' of a sequence of interest in the target nucleic acid and a second CRISPR/Cas9 complex comprising a second polynucleotide that directs the CRISPR/Cas9 enzyme to bind 5' of the sequence of interest in the target nucleic acid.

In some embodiments, the first polynucleotide is immobilized to the solid support. In some embodiments, the first polynucleotide comprises a 3' portion comprising a CRISPR/Cas9 end sequence and a 5' portion comprising a single-stranded guide RNA (sgRNA) that directs the CRISPR/Cas9 complex may be immobilized directly or via a linker molecule coupling the CRISPR/Cas9 enzyme to the solid support.

As used herein, the term "single-stranded guide RNA" or "sgRNA" refers to single-stranded RNA capable of hybridizing to a target sequence in the target double-stranded nucleic acid of interest. The sgRNA interacts with the CRISPR/Cas9 enzyme and the target sequence (i.e., protospacer sequence) such that it guides the CRISPR/Cas9 enzyme to the target sequence at which site the CRISPR/Cas9 enzyme cleaves the target sequence.

In some embodiments, where the CRISPR/Cas9 enzymes are directed in a non-sequence specific manner, the target sequence has no sequence limitation except that the sequence is adjacent to a protospacer adjacent motif (PAM). For example, PAM sequences for Cas9 proteins include, but are not limited to, 5'-NGG, 5'-NGGNG, 5'-NNAGAAW, and 5'-ACAY. In some embodiments, where the CRISPR/Cas9 enzymes are directed in a sequence specific manner, the target sequence has a sequence limitation.

In some embodiments, the sgRNA is chosen that directs the CRISPR/Cas9 in a non-sequence specific manner, such that the CRISPR/Cas9 enzyme will cleave the nucleic acid in a random manner producing a range of fragment sizes.

In some embodiments, the sgRNA is from 10 to 40 nucleotides, 15 to 30 nucleotides, or 17 or 20 nucleotides. Examples of sgRNA include, but are not limited to, short randomers or sequences of (GC)ₙ or (AT)ₙ, wherein n is from 5 to 20, 8 to 15, or 10. Those skilled in the art are familiar with sgRNA design and construction, e.g., sgRNA design tools are available on the internet or from commercial sources. The sgRNA can be synthesized chemically, synthesized enzymatically, or a combination thereof.

In some embodiments, the first polynucleotide is "biotinylated" and the solid support comprises one or more biotin binding proteins. As used herein, the term "biotinylated" refers to the process of covalently attaching biotin. Biotin binding proteins are well known to those of ordinary skill in the art and include, but are not limited to, avidin, streptavidin, neutravidin, anti-biotin antibodies, biotin receptors, and biotin-binding enzymes, such as biotinidase, biotin holocarboxylase synthetase, etc.

In some embodiments, one or more reagents that reduce the nucleic acid binding specificity of nucleic acid binding enzymes, such as CRISPR/Cas9, may be used alone, or in combination with the sgRNA (i.e., another condition which directs the CRISPR/Cas9 enzyme to randomly fragment or cleave the target double-stranded nucleic acid). In some embodiments, the CRISPR/Cas9 enzymes are treated with the one or more reagents to reduce the nucleic acid binding specificity of the CRISPR/Cas9 enzymes and induce random fragmentation when a target double-stranded nucleic acid is applied to the solid support having the treated CRISPR/Cas9 enzymes immobilized thereon. Binding specificity reducing reagents are well known to a person of ordinary skill in the art. Examples of the binding specificity reducing agents include, but are not limited to, betaine, dimethyl sulfoxide (DMSO), ethanol, ethylene glycol, dimethylacetamide, dimethylformamide, and/or sulphalane.

In some embodiments, a condition which directs the CRISPR/Cas9 enzyme to randomly fragment or cleave the target double-stranded nucleic acid may include, in addition or in the alternative to other conditions, use of a high glycerol concentration (e.g., > 5% v/v), a high concentration of the CRISPR/Cas9 enzyme/µg of DNA (e.g. 100 units/µg), of a non-optimal buffer (e.g., with non-optimal ionic strength or pH), a prolonged reaction time, and/or use of divalent cations other than Mg²⁺ (e.g., Mn²⁺, Cu²⁺, Co²⁺, and/or Zn²⁺).

When referring to immobilization of molecules (*e.g.,* nucleic acids) to a solid support, the terms "immobilized" and "attached" are used interchangeably herein and both terms are intended to encompass direct or indirect, covalent or non-covalent attachment, unless indicated otherwise, either explicitly or by context. In certain embodiments of the invention covalent attachment may be preferred, but generally all that is required is that the molecules (*e.g.,* nucleic acids) remain immobilized or attached to the support under the conditions in which it is intended to use the support, for example in applications requiring nucleic acid amplification and/or sequencing.

Certain embodiments of the invention may make use of solid supports comprised of an inert substrate or matrix (e.g. glass slides, polymer beads, etc.) which has been functionalized, for example by application of a layer or coating of an intermediate material comprising reactive groups which permit covalent attachment to biomolecules, such as polynucleotides. Examples of such supports include, but are not limited to, polyacrylamide hydrogels supported on an inert substrate such as glass, particularly polyacrylamide hydrogels as described in WO 2005/065814 and US 2008/0280773, the contents of which are incorporated herein in their entirety by reference. In such embodiments, the biomolecules (*e.g.,* polynucleotides) may be directly covalently attached to the intermediate material (*e.g.,* the hydrogel) but the intermediate material may itself be non-covalently attached to the substrate or matrix (*e.g.,* the glass substrate). The term "covalent attachment to a solid support" is to be interpreted accordingly as encompassing this type of arrangement.

The terms "solid surface," "solid support" and other grammatical equivalents herein refer to any material that is appropriate for or can be modified to be appropriate for the attachment of the CRISPR/Cas9 enzymes and CRISPR/Cas9 complexes. In some embodiments, the solid support comprises microparticles, such as beads, a patterned surface, or wells. As will be appreciated by those of skill in the art, the number of possible substrates is very large. Possible substrates include, but are not limited to, glass and modified or functionalized glass, plastics (including, for example, acrylics, polystyrene and copolymers of styrene and other materials, polypropylene, polyethylene, polybutylene, polyurethanes, Teflon^{™}, etc.), polysaccharides, nylon or nitrocellulose, ceramics, resins, silica or silica-based materials including silicon and modified silicon, carbon, metals, inorganic glasses, plastics, optical fiber bundles, and a variety of other polymers. Particularly useful solid supports and solid surfaces for some embodiments are located within a flow cell apparatus. Exemplary flow cells are set forth in further detail below.

In some embodiments, the solid support comprises a patterned surface suitable for immobilization of CRISPR/Cas9 enzymes or complexes in an ordered pattern. A "patterned surface" refers to an arrangement of different regions in or on an exposed layer of a solid support. For example, one or more of the regions can be features where one or more CRISPR/Cas9 enzymes or complexes are present. The features can be separated by interstitial regions where CRISPR/Cas9 enzymes or complexes are not present. In some embodiments, the pattern can be an x-y format of features that are in rows and columns. In some embodiments, the pattern can be a repeating arrangement of features and/or interstitial regions. In some embodiments, the pattern can be a random arrangement of features and/or interstitial regions. In some embodiments, the CRISPR/Cas9 enzymes or complexes are randomly distributed upon the solid support. In some embodiments, the CRISPR/Cas9 enzymes or complexes are distributed on a patterned surface. Exemplary patterned surfaces that can be used in the methods and compositions set forth herein are described in US Pat. App. Publ. Nos. 2012/0316086 A1 and 2013/0116153 A1, each of which is incorporated herein by reference.

In some embodiments, the solid support comprises an array of wells or depressions in a surface. This may be fabricated as is generally known in the art using a variety of techniques, including, but not limited to, photolithography, stamping techniques, molding techniques and microetching techniques. As will be appreciated by those in the art, the technique used will depend on the composition and shape of the array substrate.

The composition and geometry of the solid support can vary with its use. In some embodiments, the solid support is a planar structure such as a slide, chip, microchip and/or array. As such, the surface of a substrate can be in the form of a planar layer. In some embodiments, the solid support comprises one or more surfaces of a flow cell. The term "flow cell" as used herein refers to a chamber comprising a solid surface across which one or more fluid reagents can be flowed. Examples of flow cells and related fluidic systems and detection platforms that can be readily used in the methods of the present disclosure are described, for example, in Bentley et al., Nature 456:53-59 (2008), WO 04/018497; US 7,057,026; WO 91/06678; WO 07/123744; US 7,329,492; US 7,211,414; US 7,315,019; US 7,405,281, and US 2008/0108082, each of which is incorporated herein by reference.

In some embodiments, the solid support or its surface is non-planar, such as the inner or outer surface of a tube or vessel. In some embodiments, the solid support comprises microspheres or beads. By "microspheres" or "beads" or "particles" or grammatical equivalents herein is meant small discrete particles. Suitable bead compositions include, but are not limited to, plastics, ceramics, glass, polystyrene, methylstyrene, acrylic polymers, paramagnetic materials, thoria sol, carbon graphite, titanium dioxide, latex or cross-linked dextrans such as Sepharose, cellulose, nylon, cross-linked micelles and Teflon^{™}, as well as any other materials outlined herein for solid supports may all be used. "Microsphere Detection Guide" from Bangs Laboratories, Fishers, Ind., is a helpful guide. In certain embodiments, the microspheres are magnetic microspheres or beads.

The beads need not be spherical; irregular particles may be used. Alternately or in addition thereto, the beads may be porous. The bead sizes range from nanometers, *i.e.,* 100 nm, to millimeters, *i.e.,* 1 mm, with beads from about 0.2 micron to about 200 microns being preferred, and from about 0.5 to about 5 micron being particularly preferred, although in some embodiments smaller or larger beads may be used.

Figure 1 generally illustrates the method according to one embodiment. CRISPR/Cas9 complexes are immobilized on a surface. The CRISPR/Cas9 complexes have a CRISPR/Cas9 enzyme ("Cas9") and a biotinylated polynucleotide comprising a 3' portion comprising a CRISPR/Cas9 end sequence and a 5' portion comprising a single-stranded guide RNA (sgRNA). A target double-stranded nucleic acid is applied to the solid support, randomly fragmented by the CRISPR/Cas9 complexes, and the double-stranded nucleic acid fragments are bound to the CRISPR/Cas9 complexes immobilized on the surface. The size of the nucleic acid fragment depends upon the distance between the immobilized CRISPR/Cas9 enzymes on the surface. For example, in some embodiments, the CRISPR/Cas9 enzymes or complexes are present on the solid support at a density of at least 10³, 10⁴, 10⁵, or at least 10⁶ complexes per mm².

When target double-stranded nucleic acid is applied to the solid support, the CRISPR/Cas9 enzymes will fragment the target double-stranded nucleic acid, thus generating randomly fragmented, double-stranded nucleic acid fragments coupled at both ends to the surface of the solid support. In some embodiments, the length of double-stranded nucleic acid fragments can be varied by changing the density of the CRISPR/Cas9 enzymes or complexes thereof on the surface. In some embodiments, the lengths of the double-stranded nucleic acid fragments in the immobilized library are proportional to the density of the CRISPR/Cas9 enzymes or complexes on the solid support. In certain embodiments, the length of the resulting double-stranded nucleic acid fragments is less than 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 10000 bp, 30000 bp or less than 100,000 bp. In such embodiments, the double-stranded nucleic acid fragments can then be amplified into clusters using standard cluster chemistry, as exemplified by the disclosure of US Patent Nos. 7,985,565 and 7,115,400, the contents of each of which is incorporated herein by reference in its entirety.

In some embodiments, the length of the templates is longer than what can be suitably amplified using standard cluster chemistry. For example, in some embodiments, the length of templates is longer than 100 bp, 200 bp, 300 bp, 400 bp, 500 bp, 600 bp, 700 bp, 800 bp, 900 bp, 1000 bp, 1100 bp, 1200 bp, 1300 bp, 1400 bp, 1500 bp, 1600 bp, 1700 bp, 1800 bp, 1900 bp, 2000 bp, 2100 bp, 2200 bp, 2300 bp, 2400 bp, 2500 bp, 2600 bp, 2700 bp, 2800 bp, 2900 bp, 3000 bp, 3100 bp, 3200 bp, 3300 bp, 3400 bp, 3500 bp, 3600 bp, 3700 bp, 3800 bp, 3900 bp, 4000 bp, 4100 bp, 4200 bp, 4300 bp, 4400 bp, 4500 bp, 4600 bp, 4700 bp, 4800 bp, 4900 bp, 5000 bp, 10000 bp, 30000 bp or longer than 100,000 bp. In particular embodiments, the length of the template can be within a range defined by an upper and lower limit selected from those exemplified above.

In certain embodiments, prior to cluster generation, the double-stranded nucleic acid fragments immobilized on the surface of the solid support can imaged. For example, an intercalating dye can be applied to at least a portion of the immobilized library of double-stranded nucleic acid fragments to obtain a set of stained immobilized fragments, which can be imaged to preserve a record of the position of the backbone of the nucleic acid molecule on the surface. Following cluster generation and sequencing, the coordinates of clusters can be associated with their position on the original backbone, thus assisting in alignment of reads along a molecule and genome assembly.

In some embodiments, the step of applying a target double-stranded nucleic acid comprises adding a biological sample to the solid support. The biological sample can be any type that comprises nucleic acid and which can be deposited onto the solid surface for fragmentation. For example, the sample can comprise nucleic acid in a variety of states of purification, including purified nucleic acid. However, the sample need not be completely purified, and can comprise, for example, nucleic acid mixed with protein, other nucleic acid species, other cellular components and/or any other contaminant. In some embodiments, the biological sample comprises a mixture of DNA, protein, other nucleic acid species, other cellular components and/or any other contaminant present in approximately the same proportion as found *in vivo.* For example, in some embodiments, the components are found in the same proportion as found in an intact cell. In some embodiments, the biological sample has a 260/280 ratio of less than 2.0, 1.9, 1.8, 1.7, 1.6, 1.5, 1.4, 1.3, 1.2, 1.1, 1.0, 0.9, 0.8, 0.7, or less than 0.60. In some embodiments, the biological sample has a 260/280 ratio of at least 2.0, 1.9, 1.8, 1.7, 1.6, 1.5, 1.4, 1.3, 1.2, 1.1, 1.0, 0.9, 0.8, 0.7, or at least 0.60. Because the methods provided herein allow nucleic acid to be bound to a solid support, any unbound nucleic acids or other contaminants can be removed merely by washing the solid support after surface bound fragmentation occurs. The biological sample can comprise, for example, a crude cell lysate or whole cells. For example, a crude cell lysate that is applied to a solid support in a method set forth herein, need not have been subjected to one or more of the separation steps that are traditionally used to isolate nucleic acids from other cellular components. Exemplary separation steps are set forth in Maniatis et al., Molecular Cloning: A Laboratory Manual, 2d Edition, 1989, and Short Protocols in Molecular Biology, ed. Ausubel, et al, hereby incorporated by reference.

Thus, in some embodiments, the biological sample can comprise, for example, blood, plasma, serum, lymph, mucus, sputum, urine, semen, cerebrospinal fluid, bronchial aspirate, feces, and macerated tissue, or a lysate thereof, or any other biological specimen comprising nucleic acid. One advantage of the methods and compositions presented herein that a biological sample can be added to the flow cell and subsequent lysis and purification steps can all occur in the flow cell without further transfer or handling steps, simply by flowing the necessary reagents into the flow cell.

Also presented herein are solid supports having a library of tagged nucleic fragments immobilized thereon prepared according to the above methods.

### II. Physical Maps of Immobilized Polynucleotide Molecules

Also presented herein are methods of generating a physical map of immobilized polynucleotides. The methods can advantageously be exploited to identify clusters likely to contain linked sequences (*i.e.,* the first and second portions from the same target polynucleotide molecule). The relative proximity of any two clusters resulting from an immobilized polynucleotide thus provides information useful for alignment of sequence information obtained from the two clusters. Specifically, the distance between any two given clusters on a solid surface is positively correlated with the probability that the two clusters are from the same target polynucleotide molecule, as described in greater detail in WO 2012/025250, which is incorporated herein by reference in its entirety.

As an example, in some embodiments, long dsDNA molecules stretching out over the surface of a flow cell are fragmented *in situ,* resulting in a line of connected dsDNA bridges across the surface of the flow cell. Further, a physical map of the immobilized DNA can then be generated. The physical map thus correlates the physical relationship of clusters after immobilized DNA is amplified. Specifically, the physical map is used to calculate the probability that sequence data obtained from any two clusters are linked, as described in the incorporated materials of WO 2012/025250.

In some embodiments, the physical map is generated by imaging the nucleic acid to establish the location of the immobilized nucleic acid molecules across a solid surface. In some embodiments, the immobilized nucleic acid is imaged by adding an imaging agent to the solid support and detecting a signal from the imaging agent. In some embodiments, the imaging agent is a detectable label. Suitable detectable labels include, but are not limited to, protons, haptens, radionuclides, enzymes, fluorescent labels, chemiluminescent labels, and/or chromogenic agents. For example, in some embodiments, the imaging agent is an intercalating dye or non-intercalating nucleic acid binding agent. Any suitable intercalating dye or non-intercalating nucleic acid binding agent as are known in the art can be used, including, but not limited to those set forth in U.S. 2012/0282617, which is incorporated herein by reference in its entirety.

In some embodiments, the immobilized double-stranded nucleic acid fragments are further fragmented to liberate a free end prior to cluster generation. Cleaving bridged structures can be performed using any suitable methodology as is known in the art, as exemplified by the incorporated materials of WO 2012/025250. For example, cleavage can occur by incorporation of a modified nucleotide, such as uracil as described in WO 2012/025250, by incorporation of a restriction endonuclease site, or by applying solution-phase CRISPR/Cas9 enzymes or complexes to the bridged nucleic acid structures, as described elsewhere herein.

In certain embodiments, a plurality of target double-stranded nucleic acid molecules is flowed onto a flow cell comprising a plurality of nano-channels, the nano-channel having a plurality of CRISPR/Cas9 enzymes or complexes immobilized thereto. As used herein, the term nano-channel refers to a narrow channel into which a long linear nucleic acid molecule is flown. In some embodiments, no more than 1, 2, 3, 4, 5, 6, 7, 8, 9, 10, 11, 12, 13, 14, 15, 16, 17, 18, 19, 20, 30, 40, 50, 60 70, 80, 90, 100, 200, 300, 400, 500, 600, 700, 800, 900 or no more than 1000 individual long strands of target nucleic acid are flowed into each nano-channel. In some embodiments the individual nano-channels are separated by a physical barrier which prevents individual long strands of target nucleic acid from interacting with multiple nano-channels. In some embodiments, the solid support comprises at least 10, 50, 100, 200, 500, 1000, 3000, 5000, 10000, 30000, 50000, 80000 or at least 100000 nano-channels. In some embodiments, CRISPR/Cas9 enzymes or complexes bound to the surface of a nano-channel fragment the nucleic acid. Contiguity mapping can then be performed, for example, by following the clusters down the length of one of these channels. In some embodiments, the long strand of target nucleic acid can be at least 0.1kb, 1kb, 2kb, 3kb, 4kb, 5kb, 6kb, 7kb, 8kb, 9kb, 10kb, 15kb, 20kb, 25kb, 30kb, 35kb, 40kb, 45kb, 50kb, 55kb, 60kb, 65kb, 70kb, 75kb, 80kb, 85kb, 90kb, 95kb, 100kb, 150kb, 200kb, 250kb, 300kb, 350kb, 400kb, 450kb, 500kb, 550kb, 600kb, 650kb, 700kb, 750kb, 800kb, 850kb, 900kb, 950kb, 1000kb, 5000kb, 10000kb, 20000kb, 30000kb, or at least 50000kb in length. In some embodiments, the long strand of target nucleic acid is no more than 0.1kb, 1kb, 2kb, 3kb, 4kb, 5kb, 6kb, 7kb, 8kb, 9kb, 10kb, 15kb, 20kb, 25kb, 30kb, 35kb, 40kb, 45kb, 50kb, 55kb, 60kb, 65kb, 70kb, 75kb, 80kb, 85kb, 90kb, 95kb, 100kb, 150kb, 200kb, 250kb, 300kb, 350kb, 400kb, 450kb, 500kb, 550kb, 600kb, 650kb, 700kb, 750kb, 800kb, 850kb, 900kb, 950kb, or no more than 1000kb in length. As an example, a flow cell having 1000 or more nano-channels with mapped immobilized fragmentation products in the nano-channels can be used to sequence the genome of an organism with short 'positioned' reads. In some embodiments, mapped immobilized fragmentation products in the nano-channels can be used resolve haplotypes. In some embodiments, mapped immobilized fragmentation products in the nano-channels can be used to resolve phasing issues.

### III. Amplification and Sequencing Immobilized Nucleic Acid Fragments

**Amplification.** The present disclosure further relates to amplification of the immobilized nucleic acid fragments produced according to the methods provided herein. The immobilized nucleic acid fragments produced by surface bound CRISPR/Cas9 enzyme mediated fragmentation can be amplified according to any suitable amplification methodology known in the art. In some embodiments, the immobilized DNA fragments are amplified on a solid support. In some embodiments, the solid support is the same solid support upon which the surface bound fragmentation occurs. In such embodiments, the methods and compositions provided herein allow sample preparation to proceed on the same solid support from the initial sample introduction step through amplification and optionally through a sequencing step.

For example, in some embodiments, the immobilized nucleic acid fragments are amplified using cluster amplification methodologies, as exemplified by the disclosures of US Patent Nos. 7,985,565 and 7,115,400, the contents of each of which is incorporated herein by reference in its entirety. The incorporated materials of US Patent Nos. 7,985,565 and 7,115,400 describe methods of solid-phase nucleic acid amplification which allow amplification products to be immobilized on a solid support in order to form arrays comprised of clusters or "colonies" of immobilized nucleic acid molecules. Each cluster or colony on such an array is formed from a plurality of identical immobilized polynucleotide strands and a plurality of identical immobilized complementary polynucleotide strands. The arrays so-formed are generally referred to herein as "clustered arrays." The products of solid-phase amplification reactions such as those described in US Patent Nos. 7,985,565 and 7,115,400 are so-called "bridged" structures formed by annealing of pairs of immobilized polynucleotide strands and immobilized complementary strands, both strands being immobilized on the solid support at the 5' end, preferably via a covalent attachment. Cluster amplification methodologies are examples of methods wherein an immobilized nucleic acid template is used to produce immobilized amplicons. Other suitable methodologies can also be used to produce immobilized amplicons from immobilized nucleic acid fragments produced according to the methods provided herein. For example, one or more clusters or colonies can be formed via solid-phase PCR whether one or both primers of each pair of amplification primers are immobilized.

In other embodiments, the immobilized nucleic acid fragments are amplified in solution. For example, in some embodiments, the immobilized nucleic acid fragments are cleaved or otherwise liberated from the solid support and amplification primers are then hybridized in solution to the liberated molecules. In other embodiments, amplification primers are hybridized to the immobilized nucleic acid fragments for one or more initial amplification steps, followed by subsequent amplification steps in solution. Thus, in some embodiments an immobilized nucleic acid template can be used to produce solution-phase amplicons.

It will be appreciated that any of the amplification methodologies described herein or generally known in the art can be utilized with universal or target-specific primers to amplify immobilized nucleic acid fragments. Suitable methods for amplification include, but are not limited to, the polymerase chain reaction (PCR), strand displacement amplification (SDA), transcription mediated amplification (TMA) and nucleic acid sequence based amplification (NASBA), as described in U.S. Patent No. 8,003,354, which is incorporated herein by reference in its entirety. The above amplification methods can be employed to amplify one or more nucleic acids of interest. For example, PCR, including multiplex PCR, SDA, TMA, NASBA and the like can be utilized to amplify immobilized nucleic acid fragments. In some embodiments, primers directed specifically to the nucleic acid of interest are included in the amplification reaction.

Other suitable methods for amplification of nucleic acids can include oligonucleotide extension and ligation, rolling circle amplification (RCA) (Lizardi et al., Nat. Genet. 19:225-232 (1998), which is incorporated herein by reference) and oligonucleotide ligation assay (OLA) (See generally U.S. Pat. Nos. 7,582,420, 5,185,243, 5,679,524 and 5,573,907; EP 0 320 308 B1; EP 0 336 731 B1; EP 0 439 182 B1; WO 90/01069; WO 89/12696; and WO 89/09835, all of which are incorporated by reference) technologies. It will be appreciated that these amplification methodologies can be designed to amplify immobilized nucleic acid fragments. For example, in some embodiments, the amplification method can include ligation probe amplification or oligonucleotide ligation assay (OLA) reactions that contain primers directed specifically to the nucleic acid of interest. In some embodiments, the amplification method can include a primer extension-ligation reaction that contains primers directed specifically to the nucleic acid of interest. As a non-limiting example of primer extension and ligation primers that can be specifically designed to amplify a nucleic acid of interest, the amplification can include primers used for the GoldenGate assay (Illumina, Inc., San Diego, CA) as exemplified by U.S. Pat. No. 7,582,420 and 7,611,869, each of which is incorporated herein by reference in its entirety.

Exemplary isothermal amplification methods that can be used in a method of the present disclosure include, but are not limited to, Multiple Displacement Amplification (MDA) as exemplified by, for example Dean et al., Proc. Natl. Acad. Sci. USA 99:5261-66 (2002) or isothermal strand displacement nucleic acid amplification exemplified by, for example U.S. Pat. No. 6,214,587, each of which is incorporated herein by reference in its entirety. Other non-PCR-based methods that can be used in the present disclosure include, for example, strand displacement amplification (SDA) which is described in, for example Walker et al., Molecular Methods for Virus Detection, Academic Press, Inc., 1995; U.S. Pat. Nos. 5,455,166, and 5,130,238, and Walker et al., Nucl. Acids Res. 20:1691-96 (1992) or hyperbranched strand displacement amplification which is described in, for example Lage et al., Genome Research 13:294-307 (2003), each of which is incorporated herein by reference in its entirety. For example, isothermal amplification methods can be used with the strand-displacing Phi 29 polymerase or Bst DNA polymerase large fragment, 5'->3' exo- for random primer amplification of genomic DNA. The use of these polymerases takes advantage of their high processivity and strand displacing activity. High processivity allows the polymerases to produce fragments that are 10-20 kb in length. As set forth above, smaller fragments can be produced under isothermal conditions using polymerases having low processivity and strand-displacing activity such as Klenow polymerase. Additional description of amplification reactions, conditions and components are set forth in detail in the disclosure of U.S. Patent No. 7,670,810, which is incorporated herein by reference in its entirety.

Another nucleic acid amplification method that is useful in the present disclosure is Tagged PCR which uses a population of two-domain primers having a constant 5' region followed by a random 3' region as described, for example, in Grothues et al. Nucleic Acids Res. 21(5):1321-2 (1993), incorporated herein by reference in its entirety. The first rounds of amplification are carried out to allow a multitude of initiations on heat denatured nucleic acid based on individual hybridization from the randomly-synthesized 3' region. Due to the nature of the 3' region, the sites of initiation are contemplated to be random throughout the genome. Thereafter, the unbound primers can be removed and further replication can take place using primers complementary to the constant 5' region.

**Sequencing.** The present disclosure further relates to sequencing of the immobilized randomly fragmented, double-stranded nucleic acid fragments produced according to the methods provided herein. The immobilized double-stranded nucleic acid fragments produced by surface bound CRISPR/Cas9 enzyme mediated fragmentation can be sequenced according to any suitable sequencing methodology, such as direct sequencing, including sequencing by synthesis, sequencing by ligation, sequencing by hybridization, nanopore sequencing and the like. In some embodiments, the immobilized nucleic acid fragments are sequenced on a solid support. In some embodiments, the solid support for sequencing is the same solid support upon which the surface bound fragmentation occurs. In some embodiments, the solid support for sequencing is the same solid support upon which the amplification occurs. In some embodiments, a first solid support is used for fragmentation and a second solid support is used for amplification and sequencing.

One preferred sequencing methodology is sequencing-by-synthesis (SBS). In SBS, extension of a nucleic acid primer along a nucleic acid template (*e.g.,* a target nucleic acid or amplicon thereof) is monitored to determine the sequence of nucleotides in the template. The underlying chemical process can be polymerization (*e.g.,* as catalyzed by a polymerase enzyme). In a particular polymerase-based SBS embodiment, fluorescently labeled nucleotides are added to a primer (thereby extending the primer) in a template dependent fashion such that detection of the order and type of nucleotides added to the primer can be used to determine the sequence of the template.

Flow cells provide a convenient solid support for housing amplified nucleic acid fragments produced by the methods of the present disclosure. One or more amplified nucleic acid fragments in such a format can be subjected to an SBS or other detection technique that involves repeated delivery of reagents in cycles. For example, to initiate a first SBS cycle, one or more labeled nucleotides, nucleic acid or DNA polymerase, etc., can be flowed into/through a flow cell that houses one or more amplified nucleic acid molecules. Those sites where primer extension causes a labeled nucleotide to be incorporated can be detected. Optionally, the nucleotides can further include a reversible termination property that terminates further primer extension once a nucleotide has been added to a primer. For example, a nucleotide analog having a reversible terminator moiety can be added to a primer such that subsequent extension cannot occur until a deblocking agent is delivered to remove the moiety. Thus, for embodiments that use reversible termination, a deblocking reagent can be delivered to the flow cell (before or after detection occurs). Washes can be carried out between the various delivery steps. The cycle can then be repeated n times to extend the primer by n nucleotides, thereby detecting a sequence of length n. Exemplary SBS procedures, fluidic systems and detection platforms that can be readily adapted for use with amplicons produced by the methods of the present disclosure are described, for example, in Bentley et al., Nature 456:53-59 (2008), WO 04/018497; US 7,057,026; WO 91/06678; WO 07/123744; US 7,329,492; US 7,211,414; US 7,315,019; US 7,405,281, and US 2008/0108082, each of which is incorporated herein by reference.

Other sequencing procedures that use cyclic reactions can be used, such as pyrosequencing. Pyrosequencing detects the release of inorganic pyrophosphate (PPi) as particular nucleotides are incorporated into a nascent nucleic acid strand (Ronaghi, et al., Analytical Biochemistry 242(1), 84-9 (1996); Ronaghi, Genome Res. 11(1), 3-11 (2001); Ronaghi et al. Science 281(5375), 363 (1998); US 6,210,891; US 6,258,568 and US. 6,274,320, each of which is incorporated herein by reference). In pyrosequencing, released PPi can be detected by being immediately converted to adenosine triphosphate (ATP) by ATP sulfurylase, and the level of ATP generated can be detected via luciferase-produced photons. Thus, the sequencing reaction can be monitored via a luminescence detection system. Excitation radiation sources used for fluorescence based detection systems are not necessary for pyrosequencing procedures. Useful fluidic systems, detectors and procedures that can be adapted for application of pyrosequencing to amplicons produced according to the present disclosure are described, for example, in WIPO Pat. App. Ser. No. PCT/US11/57111, US 2005/0191698 A1, US 7,595,883, and US 7,244,559, each of which is incorporated herein by reference.

Some embodiments can utilize methods involving the real-time monitoring of nucleic acid or DNA polymerase activity. For example, nucleotide incorporations can be detected through fluorescence resonance energy transfer (FRET) interactions between a fluorophore-bearing polymerase and γ-phosphate-labeled nucleotides, or with zeromode waveguides (ZMWs). Techniques and reagents for FRET-based sequencing are described, for example, in Levene et al. Science 299, 682-686 (2003); Lundquist et al. Opt. Lett. 33, 1026-1028 (2008); Korlach et al. Proc. Natl. Acad. Sci. USA 105, 1176-1181 (2008), the disclosures of which are incorporated herein by reference.

Some SBS embodiments include detection of a proton released upon incorporation of a nucleotide into an extension product. For example, sequencing based on detection of released protons can use an electrical detector and associated techniques that are commercially available in the Ion Torrent product line from Thermo Fisher Scientific or sequencing methods and systems described in US 2009/0026082 A1; US 2009/0127589 A1; US 2010/0137143 A1; or US 2010/0282617 A1, each of which is incorporated herein by reference. Methods set forth herein for amplifying target nucleic acids using kinetic exclusion can be readily applied to substrates used for detecting protons. More specifically, methods set forth herein can be used to produce clonal populations of amplicons that are used to detect protons.

Another useful sequencing technique is nanopore sequencing (see, for example, Deamer et al. Trends Biotechnol. 18, 147-151 (2000); Deamer et al. Acc. Chem. Res. 35:817-825 (2002); Li et al. Nat. Mater. 2:611-615 (2003), the disclosures of which are incorporated herein by reference). In some nanopore embodiments, the target nucleic acid or individual nucleotides removed from a target nucleic acid pass through a nanopore. As the nucleic acid or nucleotide passes through the nanopore, each nucleotide type can be identified by measuring fluctuations in the electrical conductance of the pore (U.S. Patent No. 7,001,792; Soni et al. Clin. Chem. 53, 1996-2001 (2007); Healy, Nanomed. 2, 459-481 (2007); Cockroft et al. J. Am. Chem. Soc. 130, 818-820 (2008), the disclosures of which are incorporated herein by reference).

Exemplary methods for array-based expression and genotyping analysis that can be applied to detection according to the present disclosure are described in US Pat. Nos.7,582,420; 6,890,741; 6,913,884 or 6,355,431 or US Pat. Pub. Nos. 2005/0053980 A1; 2009/0186349 A1 or US 2005/0181440 A1, each of which is incorporated herein by reference.

An advantage of the methods set forth herein is that they provide for rapid and efficient detection of a plurality of target nucleic acid in parallel. Accordingly, the present disclosure provides integrated systems capable of preparing and detecting nucleic acids using techniques known in the art such as those exemplified above. Thus, an integrated system of the present disclosure can include fluidic components capable of delivering amplification reagents and/or sequencing reagents to one or more immobilized DNA fragments, the system comprising components such as pumps, valves, reservoirs, fluidic lines and the like. A flow cell can be configured and/or used in an integrated system for detection of target nucleic acids. Exemplary flow cells are described, for example, in US 2010/0111768 A1 and US Ser. No. 13/273,666, each of which is incorporated herein by reference. As exemplified for flow cells, one or more of the fluidic components of an integrated system can be used for an amplification method and for a detection method. Taking a nucleic acid sequencing embodiment as an example, one or more of the fluidic components of an integrated system can be used for an amplification method set forth herein and for the delivery of sequencing reagents in a sequencing method such as those exemplified above. Alternately, an integrated system can include separate fluidic systems to carry out amplification methods and to carry out detection methods. Examples of integrated sequencing systems that are capable of creating amplified nucleic acids and also determining the sequence of the nucleic acids include, without limitation, the MiSeq^{™} platform (Illumina, Inc., San Diego, CA) and devices described in US Ser. No. 13/273,666, which is incorporated herein by reference.

### IV. Solid Supports with Immobilized CRISPR/Cas9 Complexes and Methods of Preparation

Other embodiments presented herein include solid supports, such as beads, having CRISPR/Cas9 enzymes immobilized thereon. In certain embodiments, the solid supports have CRISPR/Cas9 complexes immobilized thereon that comprise a CRISPR/Cas9 enzyme bound to a first polynucleotide that directs the CRISPR/Cas9 enzymes to bind the target nucleic acid in a non-sequence specific manner. In certain other embodiments, the solid supports have CRISPR/Cas9 complexes immobilized thereon that comprise a CRISPR/Cas9 enzyme bound to a first polynucleotide that directs the CRISPR/Cas9 enzymes to bind the target nucleic acid in a sequence specific manner. In some embodiments, the different solid supports direct the CRISPR/Cas9 enzymes to bind the target nucleic acid at different sequences. In some embodiments, a first CRISPR/Cas9 enzyme on a solid support is bound to a first polynucleotide that directs the first CRISPR/Cas9 enzyme to bind the target nucleic acid 3' of a sequence of interest, and a second CRISPR/Cas9 enzyme on the solid support is bound to a second polynucleotide that directs the second CRISPR/Cas9 enzyme to bind the target nucleic acid 5' of the sequence of interest. In some embodiments, the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner (or a sequence specific manner in a population of solid supports with targeting different sequences, optionally wherein each solid support comprises a pair of sgRNA targeting flanking regions around a sequence of interest). In some embodiments, the first polynucleotide is biotinylated and the solid support comprises one or more biotin binding proteins. The density of these surface bound CRISPR/Cas9 enzymes or complexes can vary. For example, in some embodiments, the CRISPR/Cas9 enzymes or complexes are present on the solid support at a density of at least 10³, 10⁴, 10⁵, or at least 10⁶ enzymes or complexes per mm².

### V. Fragmentation using CRISPR/Cas9 Microparticles

One embodiment presented herein is a population of microparticles (e.g., beads) having CRISPR/Cas9 enzymes or complexes immobilized thereto. The use of a solid support, such as beads, can provide several advantages over solution-based fragmentation. For example, in standard solution-based fragmentation, it is difficult to control the final fragment size of the fragmentation reaction. Fragment size is a function of the ratio of CRISPR/Cas9s to the amount and size of nucleic acid and to the duration of the reaction. Even if these parameters are controlled, size selection fractionation is commonly required as an additional step to remove excess small fragments shorter than the combined paired-read lengths. The methods provided herein avoid those disadvantages. Specifically, bead-immobilized CRISPR/Cas9 enzymes or complexes allow for selection of final fragment size as a function of the spatial separation of the bound CRISPR/Cas9 enzymes or complexes, independent of the quantity of CRISPR/Cas9 beads added to the fragmentation reaction. An additional limitation of solution-based fragmentation is that it is typically necessary to do some form of purification of the products of the fragmentation reaction both before and after PCR amplification. This typically necessitates some transfer of reactions from tube to tube. In contrast, fragmentation products on the bead based CRISPR/Cas9s can be washed and later released for amplification or other downstream processing, thus avoiding the need for sample transfer. For example, in embodiments where CRISPR/Cas9s enzymes or complexes are assembled on paramagnetic beads, purification of the fragmentation reaction products can easily be achieved by immobilizing the beads with a magnets and washing. Thus, in some embodiments, fragmentation and other downstream processing such as PCR amplification can all be performed in a single tube, vessel, droplet or other container. In some embodiments, fragmentation and downstream processing of samples takes place on a microfluidic droplet based device, as described in U.S. Publ. No. 2013/0116128, which is incorporated herein by reference in its entirety. For example, in a microfluidic droplet based device, a droplet containing target nucleic acid, wash buffer or other reagents may be passed over a surface comprising immobilized CRISPR/Cas9 enzymes or complexes. Likewise, a droplet comprising beads having CRISPR/Cas9s enzymes or complexes immobilized thereon may be contacted with target double-stranded nucleic acid, wash buffer or other reagents in a microfluidic droplet based device.

When the CRISPR/Cas9 beads are added to a solution of target double-stranded nucleic acid in a fragmentation buffer, random fragmentation takes place, linking the target double-stranded nucleic acid to the surface of the beads. An immobilized library of randomly fragmented, double-stranded nucleic acid fragments is generated.

In some embodiments, the length of the bridged fragments can be dictated by the density of the CRISPR/Cas9 enzymes or complexes on the surface of the bead. Once fragmentation is complete, the double-stranded nucleic acid fragments can be liberated from the surface of the bead using any suitable method. In some embodiments, the fragmentation products are liberated from the beads using an amplification method such as suppression PCR, step-out PCR and the like. In some embodiments, the fragmentation products are liberated from the beads by cleavage. The cleavage can be, for example, chemical, enzymatic, photochemical or a combination thereof. It will be appreciated that any suitable method for releasing one or more fragmentation products from a solid support can be utilized in the methods provided herein.

Nucleic acids can be efficiently contacted with surface bound CRISPR/Cas9 enzymes or complexes using any suitable method for increasing the probability of contact. For example, in some embodiments, precipitation of nucleic acid onto the solid surface can be utilized to increase contact between the target double-stranded nucleic acid and the CRISPR/Cas9 enzymes or complexes on the solid surface. Any one of a number of methods that are known in the art for contacting nucleic acids with a solid support can be utilized, as exemplified by the disclosure of WO 2010/115122, which is incorporated by reference in its entirety. As will be appreciated by one of skill in the art, nucleic acid can be precipitated onto a solid support by the addition of PEG, ethanol or any one of a variety of other agents known to precipitate nucleic acid onto surfaces, including, for example, any one of a number of buffers used in solid phase reversible immobilization (SPRI) technology.

In some embodiments, a population of beads bearing immobilized CRISPR/Cas9 enzymes or complexes can be mixed with an excess of beads that bear no CRISPR/Cas9s enzymes, complexes, or oligonucleotides, thereby reducing the likelihood of fragmentation across two or more different beads. Another method to reduce the likelihood of fragmentation across two or more different beads includes immobilizing beads so contact between beads is minimized. Immobilization of beads can be accomplished by any of a number of techniques known in the art, including, for example, immobilizing the beads via magnetism to the sides of a solid surface such as a microcentrifuge tube, or any other immobilization technique as exemplified by the incorporated materials of WO 2010/115122.

In some embodiments, CRISPR/Cas9 beads can be used to isolate and identify nucleic acids from a single cell, such as a prokaryotic or eukaryotic cell. For example, in some embodiments, particles such as beads are coated with indexed CRISPR/Cas9 enzymes or complexes which share the same index (all of the CRISPR/Cas9s enzymes or complexes present on a particular bead carry the same index, which is different from the index present on another bead). The beads can then be placed inside cells through any one of variety of methodologies known in the art. For example, methods for delivering beads inside cells include, but are not limited to gene guns, photothermal nanoblades (Wu et al. Anal Chem. (2011) 4:1321-7), and peptides used in conjunction with cell permeabilizing substances (Nitin et al Ann Biomed Eng. (2009) 37:2018-2027) and the like. It will be appreciated that any suitable method for associating nucleic acid from a single cell with a particle bearing indexed CRISPR/Cas9 enzymes or complexes can be used in the methods presented herein.

In some embodiments, CRISPR/Cas9 enzymes or complexes can be covalently attached to the beads as described in detail hereinabove. In addition, or in the alternative, CRISPR/Cas9 enzymes or complexes can be released from the beads upon the application of a chemical or physical stimulus. Some examples of stimuli which can trigger release of CRISPR/Cas9 enzymes or complexes from a solid support include light and/or temperature changes (e.g., heat). In some embodiments, the CRISPR/Cas9 enzymes or complexes are released from the solid support using the activity of an enzyme such as a restriction endonuclease. In certain embodiments, the CRISPR/Cas9 enzymes or complexes can be detached from the beads and move freely inside the cell. Once the beads (or alternatively, the released CRISPR/Cas9 enzymes or complexes) come into contact with chromatin or nucleic acid, fragmentation can take place. It will be understood that in eukaryotic and prokaryotic systems, not all genomic DNA will always be accessible and/or available for fragmentation. In some embodiments, up to 0.001%, 0.01%, 0.1%, 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 95% or 99% or more that 99% of the total nucleic acid in a cell is fragmented by the CRISPR/Cas9 enzymes or complexes.

In some embodiments, the double-stranded nucleic acid fragments are tagged, for example with a first tag comprising a first tag domain. In some embodiments, the first tag domain can comprise a region for cluster amplification and/or a region for priming a sequencing reaction. Tagging of the fragments makes it possible to identify reads from the same cell or other biological sample by grouping together reads that share the same tag. These reads can be considered as derived from the same solid support or bead (and therefore from the same cell or biological sample).

In some embodiments, an approach can be used to ensure that an individual biological sample or target double-stranded nucleic acid is not fragmented by multiple solid supports or beads. For example, one approach is to use beads of a size which is similar to that of the cell. This would ensure that a cell would not be able to accommodate multiple beads. In addition, or in the alternative, another approach is to make use of a cell to bead ratio which favors single cell targeting. For example, if there are far more cells than beads, then the Poisson distribution of beads inside the cells means that the cells that have taken up a single bead far outnumber the cells that have taken up two or more beads.

In some embodiments, the single cell approach described above can be used to determine whether two single nucleotide polymorphisms (SNPs) or structural rearrangements are present in the same cell. For example, in the case of heterogeneous populations of cancer cells, knowing whether two SNPs are present in the same cell or in different cells could aid in implementing the right cancer therapy.

In some embodiments, the single cell approach described above can be used to study RNA. For example, by coating the beads with suitable enzymes (i.e., reverse transcriptase) and oligonucleotides for a reverse transcription step, gene expression at the single cell level can be analyzed. In one embodiment, after introducing the beads coated with reverse transcriptase, oligonucleotides and CRISPR/Cas9 enzymes, the cytoplasmic RNA can be converted into cDNA, tagged and prepared inside the cells.

### VI. Methods of Assembling Long Reads using Immobilized CRISPR/Cas9 Enzymes

Assembly of nucleic acid fragments enables isolation of individual long nucleic acid molecules within a population of nucleic acid molecules and conversion of each nucleic acid into a fragment library. When the library of nucleic acid fragments is tagged and sequenced, the nucleic acid fragments can be assembled back into their original long molecule, for example, by reference to the tags they contain.

Target double-stranded nucleic acid can be efficiently contacted with surface bound CRISPR/Cas9s using any suitable method for increasing the probability of contact as discussed hereinabove.

The methods can be performed using any one of a variety of known formats, for example, with a combination of fragmentation reagents and a bead array for the library preparation, followed by an indexed sequencing run and bespoke data analysis. Any other suitable method that maintains beads in static separation from one another can be used for surface fragmentation and indexing of samples. For example, physical configurations such as wells or small depressions in the substrate that can retain the beads, such that a microsphere can rest in the well, or the use of other forces (magnetic or compressive), or chemically altered or active sites, such as chemically functionalized sites, electrostatically altered sites, hydrophobically and/or hydrophilically functionalized sites, or spots of adhesive.

In some embodiments, the microspheres are non-covalently associated in the wells, although the wells may additionally be chemically functionalized as is generally described below, cross-linking agents may be used, or a physical barrier may be used, e.g., a film or membrane over the beads.

In certain embodiments, the surface of the substrate is modified to contain chemically modified sites that can be used to attach, either covalently or non-covalently, the microspheres of the invention to the discrete sites or locations on the substrate. "Chemically modified sites" in this context includes, but is not limited to, the addition of a pattern of chemical functional groups including amino groups, carboxy groups, oxo groups and thiol groups, that can be used to covalently attach microspheres, which generally also contain corresponding reactive functional groups; the addition of a pattern of adhesive that can be used to bind the microspheres (either by prior chemical functionalization for the addition of the adhesive or direct addition of the adhesive); the addition of a pattern of charged groups (similar to the chemical functionalities) for the electrostatic attachment of the microspheres, e.g., when the microspheres comprise charged groups opposite to the sites; the addition of a pattern of chemical functional groups that renders the sites differentially hydrophobic or hydrophilic, such that the addition of similarly hydrophobic or hydrophilic microspheres under suitable experimental conditions will result in association of the microspheres to the sites on the basis of hydro affinity. For example, the use of hydrophobic sites with hydrophobic beads, in an aqueous system, drives the association of the beads preferentially onto the sites. As outlined above, "pattern" in this sense includes the use of a uniform treatment of the surface to allow attachment of the beads at discrete sites, as well as treatment of the surface resulting in discrete sites. As will be appreciated by those in the art, this may be accomplished in a variety of ways.

In some embodiments, when a target double-stranded nucleic acid is applied to the solid support, the target double-stranded nucleic acid gets randomly fragmented many times by the CRISPR/Cas9 enzymes or complexes on the solid support. Each fragment becomes immobilized to the solid support. In some embodiments, the solid support is a bead and the physical separation of beads in the array chip prevents a nucleic acid molecule from reaching between two beads. In other embodiments, the beads are in close contact and one or more nucleic acid molecules may stretch between two or more solid supports. In some embodiments, more than one target double-stranded nucleic acid molecule can be fragmented per solid support. The probability of two alleles being fragmented onto the same solid support or bead is low and is a function of the concentration of the nucleic acid added, and the number of solid supports or beads. For example, to avoid two alleles occurring in the same well, 0.1x haplome equivalents (50,000 genomes equivalents) would need to be loaded to 1 million beads.

In some embodiments, the double-stranded nucleic acid fragments are tagged by any of the known methods for tagging. The double-stranded nucleic acid fragments may be tagged, for example, with a first tag comprising a first tag domain. In some embodiments, the first tag domain comprises a region for priming a sequencing reaction. Once the fragmentation, and optionally tagging, is complete the double-stranded nucleic acid fragment can be transferred from the surface of the solid support to solution so that individual double-stranded nucleic acid fragments can be pooled and prepared for the next steps, such as sequencing.

Release or liberation of the immobilized double-stranded nucleic acid fragments from the solid support to the solutions can be achieved using any suitable methodology as is known in the art. For example, in some embodiments, the fragmented molecules can be liberated by cleaving off the surface of the beads via, for example, a cleavage moiety present at the 5' end of the surface bound oligonucleotides. The cleavage moiety can be any moiety suitable for cleavage of a nucleic acid strand from a solid support. Examples of methods that utilize a cleavage moiety include, but are not limited to, restriction endonuclease cleavage, chemical cleavage, RNase cleavage, photochemical cleavage, and the like, including those cleavage methods and cleavage moieties set forth in WO 2006/064199, which is incorporated by reference in its entirety.

Tags may include "primer" regions that can be used as hybridization points to hybridize PCR step-out primers that enable additional sequences to be added such as amplification and sequencing primers. For example, amplification primers P5 and P7 can be added. Once added, suppression PCR can be used, for example, to enrich for molecules that have P5 adaptors on one end and P7 on the other. In some embodiments, amplification can be performed directly off the solid support (e.g., beads) with step-out primers that add P5 and P7 adaptor sequences by suppression PCR.

In another embodiment, each solid support can have two types of surface grafted oligonucleotides where the primer sequence is either P5-Read1 sequencing primer or P7-Read 2 sequencing primer. This will result in mixed P5-P7 CRISPR/Cas9 enzymes or complexes. These can either be cleaved off the solid support and followed by suppression PCR to enrich the P5/P7 molecules or amplified directly off the solid support, as described above.

### EQUIVALENTS

The foregoing written specification is considered to be sufficient to enable one skilled in the art to practice the embodiments. The foregoing description and Examples detail certain embodiments and describes the best mode contemplated by the inventors. It will be appreciated, however, that no matter how detailed the foregoing may appear in text, the embodiment may be practiced in many ways and should be construed in accordance with the appended claims and any equivalents thereof.

The term "comprising" is intended herein to be open-ended, including not only the recited elements, but further encompassing any additional elements.

As used herein, the term "about" refers to a numeric value, including, for example, whole numbers, fractions, and percentages, whether or not explicitly indicated. The term about generally refers to a range of numerical values (e.g., +/-5-10% of the recited range) that one of ordinary skill in the art would consider equivalent to the recited value (e.g., having the same function or result). When terms such as at least and about precede a list of numerical values or ranges, the terms modify all of the values or ranges provided in the list. In some instances, the term about may include numerical values that are rounded to the nearest significant figure.

Throughout this application various publications, patents and/or patent applications have been referenced. The disclosure of these publications in their entireties is hereby incorporated by reference in this application.

### Summary Paragraphs

Paragraph 1. A method of preparing an immobilized library of randomly fragmented, double-stranded nucleic acid fragments comprising:
   (a) providing a solid support having CRISPR/Cas9 enzymes immobilized thereon; and
   (b) applying a target double-stranded nucleic acid to the solid support under conditions whereby the target double-stranded nucleic acid is randomly fragmented by the CRISPR/Cas9 enzymes, and the CRISPR/Cas9 binds at least one strand of the double-stranded nucleic acid fragments; thereby producing an immobilized library of randomly fragmented, double-stranded nucleic acid fragments.
Paragraph 2. The method of paragraph 1, wherein the target double-stranded nucleic acid comprises double-stranded DNA (dsDNA), double-stranded RNA (dsRNA), or a double-stranded RNA/DNA hybrid.
Paragraph 3. The method of paragraph 1, wherein the target double-stranded nucleic acid is dsDNA.
Paragraph 4. The method of any one of paragraphs 1 to 3, wherein CRISPR/Cas9 enzymes are bound to a first polynucleotide that directs the CRISPR/Cas9 enzymes to bind the target double-stranded nucleic acid in a non-sequence specific manner.
Paragraph 5. The method of paragraph 4, wherein the first polynucleotide is immobilized to the solid support.
Paragraph 6. The method of paragraph 4 or paragraph 5, wherein the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner.
Paragraph 7. The method of paragraph 6, wherein the sgRNA comprises (GC)ₙ or (AT)ₙ, wherein n is 5-20.
Paragraph 8. The method of paragraph 7, wherein n is 10.
Paragraph 9. The method of paragraph 6, wherein the sgRNA is 10 to 40 nucleotides.
Paragraph 10. The method of paragraph 9, wherein the sgRNA is 17 or 20 nucleotides.
Paragraph 11. The method of any one of paragraphs 4 to 10, wherein the first polynucleotide is biotinylated and the solid support comprises one or more biotin binding proteins.
Paragraph 12. The method of paragraph 11, wherein the biotin binding proteins comprise avidin, streptavidin, neutravidin, an anti-biotin antibody, a biotin receptor, and/or a biotin-binding enzyme.
Paragraph 13. The method of paragraph 12, wherein the biotin-binding enzyme comprises biotinidase or biotin holocarboxylase synthetase.
Paragraph 14. The method of any one of paragraphs 1 to 13, comprising washing the solid support with the double-stranded nucleic acid fragments immobilized thereon to remove any unbound nucleic acids.
Paragraph 15. The method of any one of paragraphs 4 to 14, comprising amplifying the double-stranded nucleic acid fragments immobilized on the solid surface.
Paragraph 16. The method of paragraph 15, wherein the amplifying comprises providing a polymerase and an amplification primer corresponding to a portion of the first polynucleotide.
Paragraph 17. The method of any one of paragraphs 1 to 16, wherein applying the target double-stranded nucleic acid to the solid support comprises treating the CRISPR/Cas9 enzymes with one or more reagents that reduce the nucleic acid binding specificity of the CRISPR/Cas9 enzymes.
Paragraph 18. The method of paragraph 18, wherein the one or more reagents comprise betaine, dimethyl sulfoxide (DMSO), ethanol, ethylene glycol, dimethylacetamide, dimethylformamide, and/or sulphalane.
Paragraph 19. The method of any one of paragraphs 1 to 18, wherein the CRISPR/Cas9 enzymes are present on the solid support at a density of at least 10³, 10⁴, 10⁵, or 10⁶ enzymes per mm².
Paragraph 20. The method of any one of paragraphs 1 to 19, wherein the lengths of the double-stranded nucleic acid fragments in the immobilized library are proportional to the density of CRISPR/Cas9 enzymes on the solid support.
Paragraph 21. The method of any one of paragraphs 1 to 20, wherein the solid support comprises microparticles, a patterned surface, or wells.
Paragraph 22. The method of paragraph 21, wherein the microparticles are beads.
Paragraph 23. The method of any one of paragraphs 1 to 22, comprising: (c) applying an intercalating dye to at least a portion of the immobilized library of double-stranded nucleic acid fragments to obtain a set of stained immobilized fragments; and obtaining an image of the stained immobilized fragments.
Paragraph 24. The method of any one of paragraphs 1 to 23, wherein applying a target double-stranded nucleic acid comprises adding a biological sample to the solid support.
Paragraph 25. The method of paragraph 24, wherein the biological sample comprises a cell lysate.
Paragraph 26. The method of paragraph 24, wherein the biological sample comprises whole cells.
Paragraph 27. The method of paragraph 24, wherein the biological sample is selected from the group consisting of blood, plasma, serum, lymph, mucus, sputum, urine, semen, cerebrospinal fluid, bronchial aspirate, feces, and macerated tissue.
Paragraph 28. The method of any one of paragraphs 1 to 27, comprising tagging the double-stranded nucleic acid fragments.
Paragraph 29. The method of paragraph 28, wherein the double-stranded nucleic acid fragments are tagged with a first tag comprising a first tag domain.
Paragraph 30. The method of paragraph 29, wherein first tag domain comprises a region for cluster amplification.
Paragraph 31. The method of paragraph 29 or paragraph 30, wherein the first tag domain comprises a region for priming a sequencing reaction.
Paragraph 32. The method of any one of paragraphs 1 to 31, comprising liberating the immobilized double-stranded nucleic acid fragments from the solid support.
Paragraph 33. The method of paragraph 32, wherein the liberating comprises cleavage of the CRISPR/Cas9 enzymes from the solid support.
Paragraph 34. The method of paragraph 32, wherein the liberating comprises performing polymerase chain reaction (PCR), strand displacement amplification (SDA), transcription mediated amplification (TMA) and nucleic acid sequence based amplification (NASBA), or other amplification process.
Paragraph 35. The method of paragraph 34, wherein the PCR comprises suppression PCR.
Paragraph 36. The method of paragraph 32, wherein the liberating comprises applying light or heat.
Paragraph 37. A method of preparing an immobilized library of randomly fragmented, double-stranded nucleic acid fragments comprising:
   (a) providing a solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner, and wherein the biotinylated first polynucleotide is bound to a biotin binding protein on the solid support; and
   (b) applying a target double-stranded nucleic acid to the solid support under conditions whereby the target double-stranded nucleic acid is randomly fragmented by the CRISPR/Cas9 complexes, and the CRISPR/Cas9 complexes bind at least one strand of the double-stranded nucleic acid fragments; thereby producing an immobilized library of randomly fragmented, double-stranded nucleic acid fragments.
Paragraph 38. The method of paragraph 37, wherein the biotin binding protein comprises avidin, streptavidin, neutravidin, an anti-biotin antibody, a biotin receptor, and/or a biotin-binding enzyme.
Paragraph 39. The method of paragraph 38, wherein the biotin-binding enzyme comprises biotinidase or biotin holocarboxylase synthetase.
Paragraph 40. The method of paragraph 38 or paragraph 39, wherein applying the target double-stranded nucleic acid to the solid support comprises treating the CRISPR/Cas9 enzymes with one or more reagents that reduce the nucleic acid binding specificity of the CRISPR/Cas9 enzymes.
Paragraph 41. The method of paragraph 40, wherein the one or more reagents comprise betaine, dimethyl sulfoxide (DMSO), ethanol, ethylene glycol, dimethylacetamide, dimethylformamide, and/or sulphalane.
Paragraph 42. The method of any one of paragraphs 37 to 41, comprising tagging the double-stranded nucleic acid fragments.
Paragraph 43. The method of paragraph 42, wherein the double-stranded nucleic acid fragments are tagged with a first tag comprising a first tag domain.
Paragraph 44. The method of paragraph 43, wherein first tag domain comprises a region for cluster amplification.
Paragraph 45. The method of paragraph 43 or paragraph 44, wherein the first tag domain comprises a region for priming a sequencing reaction.
Paragraph 46. The method of any one of paragraphs 37 to 45, comprising liberating the immobilized double-stranded nucleic acid fragments from the solid support.
Paragraph 47. The method of paragraph 46, wherein the liberating comprises cleavage of the CRISPR/Cas9 enzymes from the solid support.
Paragraph 48. The method of paragraph 47, wherein the liberating comprises performing PCR or other amplification process.
Paragraph 49. The method of paragraph 48, wherein the PCR comprises suppression PCR.
Paragraph 50. The method of paragraph 48, wherein the liberating comprises applying light or heat.
Paragraph 51. A solid support having a library of double-stranded nucleic acid fragments immobilized thereon prepared according to the method of any one of paragraphs 1 to 45.
Paragraph 52. A solid support having CRISPR/Cas9 complexes immobilized thereon, wherein the CRISPR/Cas9 complexes comprise CRISPR/Cas9 enzymes that randomly fragment a target double-stranded nucleic acid.
Paragraph 53. The solid support of paragraph 52, wherein the target double-stranded nucleic acid comprises double-stranded DNA (dsDNA), double-stranded RNA (dsRNA), or a double-stranded RNA/DNA hybrid.
Paragraph 54. The solid support of paragraph 53, wherein the target double-stranded nucleic acid is dsDNA.
Paragraph 55. The solid support of any one of paragraphs 51 to 54, wherein CRISPR/Cas9 enzymes are bound to a first polynucleotide that directs the CRISPR/Cas9 enzymes to bind the target double-stranded nucleic acid in a non-sequence specific manner.
Paragraph 56. The solid support of paragraph 55, wherein the first polynucleotide is immobilized to the solid support.
Paragraph 57. The solid support of paragraph 55 or paragraph 56, wherein the first polynucleotide comprises a 3' portion comprising a CRISPR/Cas9 end sequence and a 5' portion comprising a single-stranded guide RNA (sgRNA) that directs the CRISPR/Cas9 enzymes to bind the target nucleic acid in a non-sequence specific manner.
Paragraph 58. The solid support of paragraph 57, wherein the sgRNA comprises (GC)ₙ or (AT)ₙ, wherein n is 5-20.
Paragraph 59. The method of paragraph 58, wherein n is 10.
Paragraph 60. The method of paragraph 57, wherein the sgRNA is 10 to 40 nucleotides.
Paragraph 61. The method of paragraph 60, wherein the sgRNA is 17 or 20 nucleotides.
Paragraph 62. The solid support of any one of paragraphs 55 to 51, wherein the first polynucleotide is biotinylated and the solid support comprises biotin binding proteins.
Paragraph 63. The solid support of paragraph 62, wherein the biotin binding proteins comprise avidin, streptavidin, neutravidin, an anti-biotin antibody, a biotin receptor, and/or a biotin-binding enzyme.
Paragraph 64. The solid support of paragraph 63, wherein the biotin-binding enzyme comprises biotinidase or biotin holocarboxylase synthetase.
Paragraph 65. The solid support of any one of paragraphs 52 to 64, wherein the CRISPR/Cas9 enzymes are present on the solid support at a density of at least 10³, 10⁴, 10⁵, or 10⁶ enzymes per mm².
Paragraph 66. The solid support of any one of paragraphs 52 to 65, wherein the solid support comprises microparticles, a patterned surface, or wells.
Paragraph 67. The solid support of paragraph 66, wherein the microparticles are beads.
Paragraph 68. A composition comprising the solid support of any one of paragraphs 52 to 67 and one or more reagents that reduce the nucleic acid binding specificity of the CRISPR/Cas9 enzymes.
Paragraph 69. The composition of paragraph 68, wherein the one or more reagents comprise betaine, dimethyl sulfoxide (DMSO), ethanol, ethylene glycol, dimethylacetamide, dimethylformamide, and/or sulphalane.
Paragraph 70. A solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind a target double-stranded nucleic acid in a non-sequence specific manner, and wherein the biotinylated first polynucleotide is bound to biotin binding protein on the solid support.
Paragraph 71. The solid support of paragraph 70, wherein the biotin binding protein comprises avidin, streptavidin, neutravidin, an anti-biotin antibody, a biotin receptor, and/or a biotin-binding enzyme.
Paragraph 72. The solid support of paragraph 71, wherein the biotin-binding enzyme comprises biotinidase or biotin holocarboxylase synthetase.
Paragraph 73. The solid support of any one of paragraphs 70 to 72, wherein the CRISPR/Cas9 complexes are present on the solid support at a density of at least 10³, 10⁴, 10⁵, 10⁶ complexes per mm².
Paragraph 74. The solid support of any one of paragraphs 70 to 73, wherein the solid support comprises microparticles, a patterned surface, or wells.
Paragraph 75. The solid support of paragraph 74, wherein the microparticles are beads.
Paragraph 76. A composition comprising the solid support of any one of paragraphs 70 to 75 and one or more reagents that reduces the nucleic acid binding specificity of the CRISPR/Cas9 enzymes.
Paragraph 77. The composition of paragraph 76, wherein the one or more reagents comprise betaine, dimethyl sulfoxide (DMSO), ethanol, ethylene glycol, dimethylacetamide, dimethylformamide, and/or sulphalane.

## Claims

**1.** A solid support having CRISPR/Cas9 complexes immobilized thereon, wherein the CRISPR/Cas9 complexes comprise a CRISPR/Cas9 enzyme bound to a biotinylated first polynucleotide comprising a 3' portion comprising a CRISPR/Cas9 end sequence and a 5' portion comprising a single-stranded guide RNA (sgRNA) that directs the CRISPR/Cas9 enzymes to bind a target double-stranded nucleic acid in a non-sequence specific manner, and wherein the biotinylated first polynucleotide is bound to biotin binding protein on the solid support.

**2.** The solid support of claim 1, wherein the biotin binding protein comprises avidin, streptavidin, neutravidin, an anti-biotin antibody, a biotin receptor, and/or a biotin-binding enzyme.

**3.** The solid support of claim 2, wherein the biotin-binding enzyme comprises biotinidase or biotin holocarboxylase synthetase.

**4.** The solid support of any one of claims 1-3, wherein the CRISPR/Cas9 complexes are present on the solid support at a density of at least 10³, 10⁴, 10⁵, 10⁶ complexes per mm².

**5.** The solid support of any one of claims 1-4, wherein the solid support comprises microparticles, a patterned surface, or wells.

**6.** The solid support of claim 5, wherein the microparticles are beads.

**7.** A composition comprising the solid support of any one of claims 1-6 and one or more reagents that reduces the nucleic acid binding specificity of the CRISPR/Cas9 enzymes.

**8.** The composition of claim 7, wherein the one or more reagents comprise betaine, dimethyl sulfoxide (DMSO), ethanol, ethylene glycol, dimethylacetamide, dimethylformamide, and/or sulphalane.

**9.** The solid support of claim 1, wherein the sgRNA comprises (GC)ₙ or (AT)ₙ, wherein n is 5-20.

**10.** The solid support of claim 9, wherein n is 10.

**11.** The solid support of claim 1, wherein the sgRNA is 10 to 40 nucleotides.
The solid support of claim 1, wherein the sgRNA is 17 or 20 nucleotides.
The solid support of claim 1 comprising an immobilized library of randomly fragmented, double-stranded nucleic acid fragments bound to the CRISPR/Cas9 complexes.

**14.** The solid support of claim 13, wherein the randomly fragmented, double-stranded nucleic acid fragments comprise a first tag comprising a first tag domain.

**15.** The solid support of claim 14, wherein first tag domain comprises a region for cluster amplification.
